# EUROPÄISCHE PATENTANMELDUNG

(11) **EP 1 950 270 A1**
(43) Veröffentlichungstag der Anmeldung: **30.07.2008**
(21) Anmeldenummer: 08000690.1
(22) Anmeldetag: 15.01.2008
(51) Int. Cl.: C10G 11/00, B01J 38/10, C07C 4/20

(54) **Verfahren und Vorrichtung zur Regeneration eines Katalysators zur Dampf-Dealkylierung**

(30) Priorität: 26.01.2007 DE 102007004074
(71) Anmelder: Linde Aktiengesellschaft, 80807 München (DE)
(72) Erfinder: Fritz, Helmut, 81373 München (DE); Göke, Volker, 82151 Wolfrathausen (DE); Ponceau, Marianne, 80634 München (DE); Schödel, Nicole, 81477 München (DE); Wenning, Ulrike, 82049 Pullach (DE)

(57) **Zusammenfassung**

Die Erfindung beschreibt ein Verfahren sowie eine Vorrichtung zur Durchführung des Verfahrens zur Regeneration von Katalysatormaterial zur Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion), wobei sich das Katalysatormaterial in einer von außen beheizten Vorrichtung befindet. Zur Entfernung von höhermolekularen Kohlenstoffen von der Katalysatoroberfläche und damit zur Regeneration des Katalysatormaterials in mindestens einem Abschnitt der Vorrichtung, wird dieser Abschnitt von einem Gasgemisch überwiegend bestehend aus Wasserdampf und Wasserstoff durchströmt, während mindestens ein anderer Abschnitt der Vorrichtung zur Dampf-Dealkylierung der Kohlenwasserstofffraktion genutzt wird. Die Abschnitte der Vorrichtung weisen jeweils separate Zuführungen für die Einsatzstoffe und Abführungen für die Reaktionsprodukte auf.

## Beschreibung

Die Erfindung betrifft ein Verfahren zur Regeneration von Katalysatormaterial zur Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer Anlage zur Erzeugung von Kohlenwasserstoffen aus kohlenwasserstoffhaltigem Einsatz, wobei sich das Katalysatormaterial in einer von außen beheizten Vorrichtung befindet.

In einer Anlage zur Erzeugung von Kohlenwasserstoffen aus kohlenwasserstoffhaltigem Einsatz entsteht ein Gemisch aus Kohlenwasserstoffen mit einer unterschiedlichen Anzahl von Kohlenstoffatomen und unterschiedlichen molekularen Konfigurationen. Dieses Gemisch wird in der Anlage zur Erzeugung von Kohlenwasserstoffen aus kohlenwasserstoffhaltigem Einsatz in die einzelnen Kohlenwasserstoffprodukte aufgetrennt.

Abhängig von der jeweiligen Trennsequenz und der Art der Anlage zur Erzeugung von Kohlenwasserstoffen aus kohlenwasserstoffhaltigem Einsatz entstehen eine Kohlenwasserstofffraktion mit mindestens sechs Kohlenstoffatomen (C₆₊-Fraktion) und/oder eine Kohlenwasserstofffraktion mit mindestens sieben Kohlenstoffatomen (C₇₊-Fraktion). Diese Fraktionen enthalten als wirtschaftlich verwertbares Produkt Aromaten, welche als Ausgangsstoff für die Synthese zahlreicher Kunststoffe und zur Erhöhung der Klopffestigkeit von Benzin Verwendung finden.

Um an die wirtschaftlich verwertbaren Produkte der C₆₊-Fraktion, vor allem Benzol, zu gelangen und die Ausbeute möglichst maximal zu gestalten, wird nach dem Stand der Technik folgendes Verfahren angewendet. Die C₆₊-Fraktion wird in eine Fraktion überwiegend bestehend aus Kohlenwasserstoffen mit sechs Kohlenstoffatomen (C₆-Fraktion) und eine C₇₊-Fraktion getrennt. Aus der C₆-Fraktion kann direkt das wirtschaftlich verwertbare Produkt Benzol abgetrennt werden. Aus der C₇₊-Fraktion werden mittels einer Flüssig-Flüssig Extraktion die linearen Kohlenwasserstoffe abgetrennt und als so genanntes Raffinat weiterverarbeitet. Die von den linearen Kohlenwasserstoffen befreite C₇₊-Fraktion enthält nunmehr hauptsächlich Aromaten mit sieben bis acht Kohlenstoffatomen und wird in eine Fraktion überwiegend bestehend aus Kohlenwasserstoffen mit sieben Kohlenstoffatomen (hauptsächlich Toluol) und in eine Fraktion überwiegend bestehend aus Kohlenwasserstoffen mit acht Kohlenstoffatomen (hauptsächlich Xylol) getrennt. Die Fraktion überwiegend bestehend aus Kohlenwasserstoffen mit acht Kohlenstoffatomen wird als Einsatzstoff in ein Verfahren zur para-Xylol Gewinnung geführt. Die Fraktion überwiegend bestehend aus Kohlenwasserstoffen mit sieben Kohlenstoffatomen wird als Einsatz in ein Verfahren zur Hydro-Dealkylierung geführt wird.

Ein derartiges Verfahren zur Hydro-Dealkylierung ist zum Beispiel in W02005071045 beschrieben. Die Kohlenwasserstoffe werden in Anwesenheit eines Katalysators bei einer Temperatur von 400°C bis 650°C und einem Druck zwischen 20 bar und 40 bar mit Wasserstoff kontaktiert, wobei der Wasserstoff in einem molaren Überschuss vom drei- bis sechsfachen der Kohlenwasserstoffe vorliegt. Unter diesen Bedingungen werden die Alkylgruppen von den jeweiligen alkylierten Aromaten (wie zum Beispiel Toluol) abgespalten, so dass sich Benzol und die jeweiligen Alkane (zum Beispiel Methan) bilden.

Der Verbrauch von Wasserstoff bei der Hydro-Dealkylierung der Kohlenwasserstoffe wirkt sich negativ auf die Wirtschaftlichkeit dieses Verfahrens nach dem Stand der Technik zur Benzolgewinnung aus.

In einem alternativen Verfahren werden die C₆₊-Fraktion und/oder die C₇₊-Fraktion einer Dampf-Dealkylierung unterzogen (siehe zum Beispiel eingereichte Patentschriften DE102006058528.3 oder DE102006058529.1). Bei einer Dampf-Dealkylierung der C₆₊-Fraktion und/oder C₇₊-Fraktion entstehen hauptsächlich die beiden verwertbaren Produktstoffe Benzol und Wasserstoff neben Reaktionsprodukten wie Kohlenmonoxid und Kohlendioxid. Als Einsatzstoff der Dampf-Dealkylierung wird somit lediglich kostengünstiger Wasserdampf bei gleichzeitiger Produktion des gewünschten Wertproduktes Benzol und des wertvollen Nebenproduktes Wasserdampf benötigt.

In diesem Verfahren nach dem Stand der Technik reagieren die Kohlenwasserstoffe aus der jeweiligen Kohlenwasserstofffraktion mit Wasserdampf in der Gasphase an einem festen Katalysator. Dabei werden die Kohlenwasserstoffe und der Wasserdampf in einer Vorrichtung, zum Beispiel Rohre, an dem festen Katalysator vorbeigeführt, wobei sich der Katalysator im Inneren der Vorrichtung, zum Beispiel im Rohrinneren, befindet. Die für die Dealkylierung nötige Reaktionswärme wird der Vorrichtung von außen zugeführt, zum Beispiel durch Verbrennung der gasförmigen Reaktionsnebenprodukte Kohlenmonoxid und Methan mit Luft. Nach dem Stand der Technik entspricht die Temperatur der Einsatzstoffe der Temperatur der Reaktionsprodukte und liegt im Bereich von 400°C bis 600°C. Das Katalysatormaterial im Inneren der Vorrichtung besteht im Wesentlichen aus einem porösen Trägermaterial wie γ-Al₂O₃, MgAl Spinell und/oder Cr₂O₃, und einer an der Oberfläche des Trägermaterials befindlichen Aktivkomponente, wie Rh mit 0.1-1.0 Gew% Beladung und/oder Pd mit 0.2-2.0 Gew% Beladung. Aus den gasförmigen Reaktionsprodukten der Dampf-Dealkylierung wird über eine Druckwechseladsorption mit vorheriger Verdichtung der wirtschaftlich verwertbare Wasserstoff von den Reaktionsnebenprodukten wie Kohlenmonoxid, Kohlendioxid und Methan getrennt.

Bei der Reaktion der Einsatzstoffe am aktiven katalytischen Zentrum des Katalysatormaterials werden zu einem bestimmten Anteil neben den eigentlichen Reaktionsprodukten auch höhermolekulare Kohlenstoffverbindungen gebildet, die teilweise als Ablagerungen auf dem Katalysatormaterial verbleiben. Die sich ablagernden höhermolekularen Kohlenstoffverbindungen blockieren die Möglichkeit zur Adsorption der Edukte auf der Katalysatoroberfläche und führen so zu einer mit der Zeit zunehmenden Deaktivierung (Verkokung). Die Verkokungsgeschwindigkeit ist dabei in erster Näherung linear zum Partialdruck der Kohlenwasserstoffe in der Gasphase.

Der Prozess der Verkokung ist reversibel. Nach dem Stand der Technik wird ein so verkoktes Katalysatormaterial in einem Abstand von mehreren Wochen von den Ablagerungen durch Abbrennen unter Zufuhr von Sauerstoff oder sauerstoffhaltigem Gas befreit und somit regeneriert. Alternativ zum Abbrennen kann das Katalysatormaterial mit einem Einsatzstoff aus Wasserdampf und Wasserstoff begast werden, wodurch die Kohlenstoffablagerungen ebenfalls vom Katalysatormaterial entfernt werden. Diese Verfahrensweise ist durch die verringerte Gefahr der Ausbildung von lokal sehr heißen Stellen schonender und wirkt sich damit positiv auf die Lebensdauer des Katalysators aus. Bei beiden Verfahren zur Regeneration des Katalysatormaterials muss die Zufuhr der Kohlenwasserstofffraktion und somit die Produktion des gewünschten Wertproduktes Benzol gestoppt werden. Diese Produktionsstopps zur Regeneration des Katalysatormaterials wirken sich negativ auf die Betriebskosten des Verfahrens aus.

Der Erfindung liegt daher die Aufgabe zugrunde, ein Verfahren sowie eine Vorrichtung zur Durchführung des Verfahrens derart auszugestalten, das Produktionsstopps zur Regeneration des Katalysatorsmaterials vermieden und die Betriebskosten der Verfahrens minimiert werden.

Die vorliegende Aufgabe wird dadurch gelöst, dass zur Entfernung von höhermolekularen Kohlenstoffen von der Katalysatoroberfläche und damit zur Regeneration des Katalysatormaterials in mindestens einem Abschnitt der Vorrichtung, dieser Abschnitt / diese Abschnitte von einem Gasgemisch überwiegend bestehend aus Wasserdampf und Wasserstoff durchströmt wird/werden, während mindestens ein anderer Abschnitt der Vorrichtung zur Dampf-Dealkylierung der Kohlenwasserstofffraktion genutzt wird, wobei die einzelnen Abschnitte jeweils separat von unterschiedlichen Einsatzstoffen anströmbar sind.

Durch die unabhängige Anströmung einzelner Abschnitte der Vorrichtung kann immer ein Teil der Vorrichtung zur Dampf-Dealkylierung genutzt werden, während in einem Teil der Vorichtung das Katalysatormaterial durch die Beaufschlagung mit einem Gasgegemisch aus Wasserdampf und Wasserstoff regeneriert wird. Somit ist für die Regenierung des Katalysatormaterials kein Produktionsstopp nötig.

Vorteilhafterweise werden die Produktstoffe des/der Abschnitts/Abschnitte, welcher/welche zur Regeneration von einem Gasgemisch überwiegend bestehend aus Wasserdampf und Wasserstoff durchströmt wird/werden, mit den Produktstoffen des/der Abschnitts/Abschnitte gemischt, welcher/welche zur Dampf-Dealkylierung der Kohlenwasserstofffraktion genutzt wird/werden. Als Produktstoffe der Dampf-Dealkylierung entstehen die dealkylierten aromatischen Kohlenwasserstoffe und die entsprechenden Alkane neben den Reaktionsprodukten Wasserstoff, Kohlenmonoxid, Kohlendioxid und Methan. Bei der Regeneration des Katalysatormaterials mit dem Gasgemisch überwiegend bestehend aus Wasserdampf und Wasserstoff werden die höhermolekularen Kohlenstoffablagerungen von der Katalysatoroberfläche abgelöst und finden sich so im Produktstrom der Regeneration wieder. Dabei entstehen durch die Vergasung der stark kohlenstoffhaltigen Ablagerungen vor allem die auch im normalen Produktstrom vorhandenen Komponenten Kohlenmonoxid, Kohlendioxid, Wasserstoff und Methan sowie in geringem Umfang weitere niedermolekulare Kohlenwasserstoffe. Die Zusammensetzung der Produktströme aus den Abschnitten ist daher ähnlich und kann einer gemeinsamen Zerlegung zugeführt werden, so dass der stochiometrische Überschuss des zur Regeneration eingesetzte Wasserstoff in der Anlage verbleibt. Aus der Wasserdampfvergasung der höhermolekularen kohlenstoffhaltigen Ablagerungen wird zusätzlicher Wasserstoff produziert.

Zweckmäßigerweise wird der Wasserstoff zur Regeneration in der Vorrichtung zur Dampf-Dealkylierung erzeugt und aus den gasförmigen Reaktionsprodukte der Dampf-Dealkylierung überwiegend bestehend aus Wasserstoff, Kohlenmonoxid, Kohlendioxid und Methan nach einem mehrstufigen Verdichtungsverfahren mittels eines Druckwechseladsorptionsverfahrens abgetrennt und zumindest teilweise zur Regeneration des Katalysatormaterials verwendet.

Bevorzugt werden die Menge Wasser und Wasserstoff im Gasgemisch zur Regeneration des Katalysatormaterials an die Betriebsbedingungen der Vorrichtung zeitlich variabel angepasst. Durch die zeitlich variabele Anpassung der Menge an Wasser und Wasserstoff an die jeweiligen Betriebsbedingungen des jeweiligen Abschnitts (Regeneration oder Dealkylierung, Druck, Temperatur) läßt sich eine optimale Regeneration bei geringem Verbrauch der Einsatzstoffe erreichen.

Vorteilhafterweise wird die Gesamtwärmeleistung der Beheizung der Vorrichtung im Falle der Regeneration von mindestens einem Abschnitt der Vorrichtung an den geänderten Wärmebedarf angepasst. In einer besonders bevorzugten Ausgestaltung der Erfindung erfolgt eine lokale Anpassung Wärmeleistung der Beheizung der Vorrichtung im Falle der Regeneration von mindestens einem Abschnitt der Vorrichtung, wobei die Wärmeleistung im Bereich des/der Abschnitts/Abschnitte, der/die regeneriert wird, reduziert wird.

Vorrichtungsseitig wird die gestellte Aufgabe dadurch gelöst, dass die Vorrichtung in mindestens zwei mit Katalysatormaterial befüllte Abschnitte unterteilt ist, die separate Zuführungen für die Einsatzstoffe und Abführungen für die Produktstoffe aufweisen, so dass die Abschnitte unabhängig voneinander von den Einsatzstoffen durchströmt werden können. Durch eine entsprechende Steuerung läßt sich somit jeweils auswählen, welche Abschnitte von den Einsatzstoffen der Dampf-Dealkylierung angeströmt und somit zur Produktbildung genutzt werden und in welchen Abschnitten das Katalysatormaterial durch die Anströmung mit dem Gasgemisch überwiegend bestehend aus Wasserdampf und Wasserstoff regeneriert wird.

Vorteilhafterweise besteht ein mit Katalysatormaterial befüllter Abschnitt aus mindestens einem Rohr, bevorzugt aus mindestens einer Rohrgruppe.

Mit der vorliegenden Erfindung gelingt es insbesondere Produktionsstopps zur Regenerierung des Katalysatormaterials zu vermeiden und somit die Betriebskosten zu minimieren. Der zusätzlich Investitionsaufwand für eine separate Anströmung der einzelnen Abschnitte der Vorrichtung ist gering.

## Patentansprüche

1. Verfahren zur Regeneration von Katalysatormaterial zur Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer Anlage zur Erzeugung von Kohlenwasserstoffen aus kohlenwasserstoffhaltigem Einsatz, wobei sich das Katalysatormaterial in einer von außen beheizten Vorrichtung befindet, **dadurch gekennzeichnet, dass** zur Entfernung von schweren und höhermolekularen Kohlenstoffen von der Katalysatoroberfläche und damit zur Regeneration des Katalysatormaterials in mindestens einem Abschnitt der Vorrichtung, dieser Abschnitt / diese Abschnitte von einem Gasgemisch überwiegend bestehend aus Wasserdampf und Wasserstoff durchströmt wird/werden, während mindestens ein anderer Abschnitt der Vorrichtung zur Dampf-Dealkylierung der Kohlenwasserstofffraktion genutzt wird, wobei die Abschnitte jeweils separate Zuführungen für die Einsatzstoffe und Abführungen für die Reaktionsprodukte aufweisen.

2. Verfahren nach Anspruch 1, **dadurch gekennzeichnet, dass** die Produktstoffe des/der Abschnitts/Abschnitte, welcher/welche zur Regeneration von einem Gasgemisch überwiegend bestehend aus Wasserdampf und Wasserstoff durchströmt wird/werden, mit den Produktstoffen des/der Abschnitts/Abschnitte gemischt werden, welcher/welche zur Dampf-Dealkylierung der Kohlenwasserstofffraktion genutzt wird/werden.

3. Verfahren nach Anspruch 1 oder 2, **dadurch gekennzeichnet, dass** der Wasserstoff zur Regeneration in der Vorrichtung zur Dampf-Dealkylierung erzeugt wird.

4. Verfahren nach einem der Ansprüche 1 bis 3, **dadurch gekennzeichnet, dass** aus den gasförmigen Reaktionsprodukte der Dampf-Dealkylierung überwiegend bestehend aus Wasserstoff, Kohlenmonoxid, Kohlendioxid und Methan nach einem mehrstufigen Verdichtungsverfahren mittels eines Druckwechseladsorptionsverfahrens der gasförmige Wasserstoff abgetrennt und zumindest teilweise zur Regeneration des Katalysatormaterials verwendet wird.

5. Verfahren nach einem der Ansprüche 1 bis 3, **dadurch gekennzeichnet, dass** die Menge Wasser und Wasserstoff im Gasgemisch zur Regeneration des Katalysatormaterials an die Betriebsbedingungen der Vorrichtung angepasst werden.

6. Verfahren nach einem der Ansprüche 1 bis 5, **dadurch gekennzeichnet, dass** die Gesamtwärmeleistung der Beheizung der Vorrichtung im Falle der Regeneration von mindestens einem Abschnitt der Vorrichtung an den geänderten Wärmebedarf zeitlich variabel angepasst wird.

7. Verfahren nach einem der Ansprüche 1 bis 6, **dadurch gekennzeichnet, dass** eine lokale Anpassung Wärmeleistung der Beheizung der Vorrichtung im Falle der Regeneration von mindestens einem Abschnitt der Vorrichtung erfolgt, wobei die Wärmeleistung im Bereich des/der Abschnitts/Abschnitte, der/die regeneriert wird, reduziert wird.

8. Vorrichtung geeignet zur Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion), die von außen beheizbar und mit einem festen Katalysatormaterial befüllt ist, **dadurch gekennzeichnet, dass** die Vorrichtung in mindestens zwei mit Katalysatormaterial befüllte Abschnitte unterteilt ist, die separate Zuführungen für die Einsatzstoffe und Abführungen für die Produktstoffe aufweisen, so dass die Abschnitte unabhängig voneinander von den Einsatzstoffen durchströmt werden können.

9. Vorrichtung nach Anspruch 8, **dadurch gekennzeichnet, dass** ein mit Katalysatormaterial befüllter Abschnitt aus mindestens einem Rohr besteht.

10. Vorrichtung nach Anspruch 8 oder 9, **dadurch gekennzeichnet, dass** ein mit Katalysatormaterial befüllter Abschnitt aus mindestens einer Rohrgruppe besteht.
